# EUROPEAN PATENT SPECIFICATION

(11) **EP 0 302 097 B1**
(45) Date of publication and mention of the grant of the patent: **28.04.1993**
(21) Application number: 88901822.2
(22) Date of filing: 13.02.1988
(51) Int. Cl.: A61K 31/52, A61K 31/525, A61K 33/30, A61K 9/22

(54) **PHARMACEUTICAL COMPOSITIONS FOR TREATING OBSTRUCTIVE AIR PASSAGE DISEASES**
PHARMAZEUTISCHE ZUSAMMENSETZUNGEN ZUR BEHANDLUNG DER VERSTOPFUNG DER LUFTWEGE
COMPOSITIONS PHARMACEUTIQUES POUR LE TRAITEMENT DE MALADIES OCCLUSIVES DES VOIES RESPIRATOIRES

(30) Priority: 16.02.1987 ZA 871092; 14.10.1987 ZA 877704
(43) Date of publication of application: 08.02.1989
(73) Proprietor: VESTA MEDICINES (PROPRIETARY) LIMITED, Micor, Johannesburg 2092 (ZA)
(72) Inventor: SERFONTEIN, Willem, Jacob, 0081 Pretoria (ZA)
(74) Representative: VOSSIUS & PARTNER
(86) International application number: EP8800107
(87) International publication number: WO8806036

(56) References cited:
- GB-A-21 645 56
- Dictionnaire Vidal, O.V.P. 1966 (Paris, FR) "Athérophylline", page 193

## Description

The present invention relates to a pharmaceutical product comprising a xanthine type drug for use in the treatment of obstructive airways diseases, e.g. in bronchial asthma therapy.

Xanthines, including theophylline and it ethylene diamino-derivative, aminophylline are used increasingly in the treatment of obstructive airways diseases, e.g. in bronchial asthma, amphysema and chronic bronchitis.

Theophylline is not related chemically to other drugs used for treating asthmatic conditions for which interference with vitamin B6 functioning in the human body was reported [P.J. Collipp et al., Ann. Allergy, 35, 93, (1975)]. Theophylline causes a significant improvement in asthma-related clinical symptoms and a reduction in dosage of bronchodilators (specified as oral bronchodilators, rectal bronchodilators, oral ephedrine, nebulisers, epinephrine injections and other medications) and cortisone required to relieve symptoms, following supplementation of the treatment with a daily intake of pyridoxine (PN, the form in which vitamin B6 is generally incorporated in pharmaceutical preparations). However, Reynolds and Natta (loc. cit.) reported only "sporadic and non-uniform elevation of plasma PLP in asthmatics compared to controls when both groups are supplemented with large doses of vitamin B6 in the form of PN. (The asthmatics were "treated with a variety of bronchodilators and were under the general care of a physician".)

Reports dealing with theophylline treatment of asthmatic patients and animal experiments did not distinguish between "theophylline" and aminophylline and used the term "theophylline" when in fact aminophylline was used. Aminophylline, the ethylene diamino derivative of theophylline, as opposed to theophylline, due to the presence of the ethylene diamino moiety, reacts chemically with the biologically active aldehyde forms of vitamin B6, pyridoxal (PL) and phosphorylated pyridoxal (PLP) by forming Schiff bases which are irreversibly transformed into aldamine complexes. This results in Vitamin B6 depletion and adverse clinical effects. Supplementation by intravenous administration of PLP after stopping the administration of aminophylline reversed the adverse clinical effects in dogs and rabbits. Similarly, "aminophylline toxicity in vitamin B6 deficient animals was enhanced while it was reduced after administration of vitamin B6.

Other ethylene diamino derivatives are piperazine derivatives, e.g. piperazine acetate complex of theophylline in which the piperazine (which is released in vivo) contains a cyclic ethylene diamino moiety. This results similarly in the deactivation of PL and PLP. In the past this effect was not appreciated, and these ethylene diamine or piperazine derivatives were given preference because of their ready solubilities in water.

Because theophylline itself does not result in the removal of PL and PLP by the above-described reaction, theophylline would appear to be preferable to aminophylline, and indeed theophylline is rapidly becoming the drug of choice in modern asthma therapy. However, surprisingly theophylline nevertheless results in typical CNS side effects, namely: depression, adverse effects on mood and memory, anxiety and tremors.

Moreover, adverse side effects not only of aminophylline but also of theophylline are severely potentiated by many drugs which may be given to asthma patients in combination with theophylline therapy, e.g. other bronchodilators, gentamycin (in case of infection), dopamine (in case of shock); digoxin; this potentiating effect may result in acute toxic effects and even death. These adverse phenomena must be considered in so serious a light that the advisability of theophylline therapy becomes questionable in the circumstances referred to.

The combination of inter alia theophylline and vitamin B6 is described in French Medical Patent Specification 50 871 M in therapeutic compositions specifically for treating cardiac complaints. No explanation is given of the therapeutic mechanism. Indeed the rationale behind the teachings of the reference is obscure, because it is said that the vitamin B6 has no effect on cardiac function and theophylline is not an accepted drug in cardiac therapy. Nothing in the document suggests any benefits to be derived in the treatment or prophylaxis of bronchial asthma or other obstructive air passage diseases.

Example 2 of the reference describes a combination of piperazine-theophylline acetate and Pyridoxin-HCl in a weight ration of 1.1. This theophylline is not used as such, but in the form of a piperazine derivative, which contains a cyclic ethylene diamino moiety. It was not realised by the authors that the ethylene diamino moiety has the adverse effects as explained above, causing chemical interference with vitamin B6 in its active PL and PLP forms. The reference also makes no distinction between different B6 vitamers, which difference, according to the present invention, is significant depending on clinical conditions and the conditions of administration.

In Dictionnaire Vidal, 1966, p. 193, the use of pyridophylline (a molecule consisting of a theophylline ethyl sulfate moiety and a pyridoxine moiety in a molar ratio of 1:1) in medicaments for the treatment of, i.a., "bronchopneumathies chroniques, dyspuées asthmatiformes, broncho-emphysèure chronique" is described.

It is an object of the present invention to counteract the side effects of xanthines and in particular theophylline in asthma and other obstructive air passage therapy. It is a further or alternative object to potentiate the beneficial effects of xanthines and in particular theophylline in such therapy to permit better therapeutic effects to be attained with a given theophylline dosage, alternatively to permit lowering the therapeutic dosage of xanthines in particular theophylline and lowering the side effects thereof.

In accordance with one aspect of the present invention a pharmaceutical product is provided as set out in the opening paragraph, which is characterized by a combination as dosage units of theophylline in its non-ethylene di-aminated form and vitamin B6 in a form and amount effective to supplement PLP in cells and plasma, wherein the weight ratio of the theophylline and the vitamin B6 in the dosage units is between 2 and 120.

Preferably the theophylline and the vitamin B6 are combined in a single dosage form, e.g. a single tablet, coated tablet, capsule, syrup, suppository or vial. However, in principle it is possible to provide the two substances in separate dosage forms, e.g. in a single package intended for a method of obstructive airways therapy, e.g. treatment of emphysema, chronic bronchitis, and especially in bronchial asthma therapy comprising the combined administration of the two substances.

Both substances, the xanthine, e.g. theophylline and vitamin B6 are used within the previously accepted conventional dosage ranges. The dosage rate for the xanthine, in particular theophylline can optionally be lowered even, due to the beneficial effect of vitamin B6 supplementation.

The preferred product, preferably for oral administration, comprises a daily dosage rate for human adults of 5 to 500 mg, preferably 10 to 200 mg of PN and 50 to 600 mg, preferably 100 to 500 mg of the theophylline; or correspondingly reduced dosage rates for children.

Since zinc ions act as a co-factor in the PL kinase phosphorylation of PL to PLP, the composition, as an important preferred feature, comprises in addition a zinc salt, for example zinc chloride, the zinc salt preferably being provided in a slow-release form. Preferably the zinc salt is provided in a slow-release form in a concentration that would ensure a daily supplement of 5 - 100 mg, preferably 10 - 20 mg, of Zn.

As an alternative the zinc may be provided in the form of a zinc-vitamin B6 coordination complex in slow-release form and may comprise the usual carriers, extenders and/or excipients.

Equally important, or even more important, if PN is used as the source of vitamin B6 is the preferred inclusion of riboflavin, to provide a daily supplement of 1 mg to 20 mg, preferably 3 - 10 mg, more particularly 5 mg. Riboflavin plays an important role in the conversion of PN into PLP. Magnesium ions are also a preferred ingredient.

The compositions according to the invention may comprise the usual carriers, extenders and/or excipients.

Preferably the dosage form or forms are intended for oral administration.

The present invention is based on a number of previously unknown observations and experiments conducted by the inventor involving mechanisms or combinations of mechanisms inapplicable to the prior art or not previously taken into account correctly or at all:
a) Surprisingly, theophylline as well (but by a mechanism not previously known and different from that known for aminophylline or piperazine derivatives) causes in humans substantial and clinically important lowering of vitamin B6 blood and cellular levels.
b) The mechanism by which theophylline depresses plasma vitamin B6 in humans is not due to Schiff base formation and aldamine complexes or equivalent reactions but rather to the inhibition of enzymes associated with vitamin B6 activation leading to low intra-cellular PLP levels, namely the enzyme PL-kinase.
c) This has direct practical clinical consequences: it means that to overcome this enzyme block, the substrate PL concentration must be increased intracellularly and not extracellularly where chemical deactivation would take place.
d) Some of the important side effects previously ascribed to theophylline toxicity are in fact not due to theophylline primarily but rather to depressed intracellular vitamin B6 levels.
e) Low vitamin B6 levels in asthmatic patients not on theophylline therapy are due to excessive production of biogenic amines.
f) At least three different mechanisms exist by means of which vitamin B6 assists the asthmatic patient namely :
   (i) altered oxygen delivery to tissues
   (ii) increased respiratory muscle contractions (increased diaphragm contractility)
   (iii) minimisation of supposedly theophylline associated side effects.
g) The abovementioned CNS side effects in patients on theophylline are mainly due to low intracellular active vitamin B6 levels. This finding was not to be expected in the light of prior art findings that theophylline does not lower plasma PLP and that on the contrary plasma PLP is sporadically increased. The latter effect, we now realise is, due to PLP being released from its albumin complexes by theophylline. However, different mechanisms including increased renal excretion prevent this PLP from directly becoming available inside the tissue cells.
h) This also explains why the side effects of theophylline are potentiated by certain drugs such as gentamycin and dopamine, which are known vitamin B6 antagonists, and by conditions leading to excessive production of biogenic amines (physiological shock, cell death in severely ill patients).
i) Even theophylline - by mechanisms different from those described above for its ethylene diamino or piperazine derivatives - antagonises vitamin B6, namely by an enzyme inhibition, in particular a kinase inhibition, and this has to be taken into account in clinical situations since the consequences of this effect are mainly manifested intracellularly and are of special significance in the long term administration of theophylline such as in the treatment of bronchial asthma and other obstructive air passage diseases.
j) PL kinase inhibition depresses not only the intracellular PLP levels, but also the ratio of PLF level to PL level in the cells. Since PN is converted into PL in the liver almost entirely where, as also in the serum and in other tissues, it is reversibly phosphorylated to PLP with the aid of PL kinase. Since PL but not PLP is capable of crossing other cell membranes, it is important for maximal effect of a vitamin B6 supplement to boost the PL substrate level inside the cells to, and maintain it at, the highest possible level. This is attained maximally by supplementing the vitamin B6 at a slow and uniform rate, particularly when the source of vitamin B6 is PN, since PN is rapidly cleared from the plasma (t½ = 12 min), while the decay of plasma PL levels, although somewhat slower, also occurs relatively rapidly (t½ = 30 - 40 min). Daily bolus doses of PN would therefore result in large plasma PL fluctuations.
k) Also, the uptake of PL from the plasma into cells is a saturable process at relatively low external (i.e. in the plasma) PL concentrations. Hence it is important to raise the PL level in the plasma and to maintain such moderately elevated levels over many hours.
l) The avoidance of excessive PN levels in the plasma also serves to avoid the generation of undesirably high PN level inside the tissue cells, where due to the action of kinase the conversion of PL into the desired intracellular PLP occurs. It is now realised or the first time that this crucial phosphorylation step inside the cells (where the intracellular PLP is vital to a number of biochemical reactions) is also inhibited by theophylline. This same kinase also results in phosphorylation of PN to PNP. PN therefore competes with PL for the available kinase. An excessive intracellular supply of PN will therefore suppress the formation of intracellular PLP even further and would thus strongly augment the already present enzyme inhibition by theophylline. There exists no known alternative route by which intracellular PLP can be generated to compensate for the resultant deficiency.
m) In contrast to previously held views, it is now realised that a patient's ability to correct any deficiencies of plasma-PLP values due to previously known mechanisms involving certain theophylline derivatives is not compromised for as long as the vitamin B6 metabolising enzymes have not been affected. However, the newly discovered inhibition of PL-kinase, caused even by theophylline itself will compromise the patient's ability to correct the intracellular deficit, both in the liver and in other cells.
n) In addition, our enzyme kinetic studies have shown that PL-kinase inhibition by theophyllin is of the non-competitive type. This means that the long-term administration of theophylline alone will gradually lead to a depletion of enzyme reserves.
o) On the basis of the new knowledge discussed above, it can now be realised that in the case of asthma patients suffering from other diseases as well, the administration of theophylline alone can be dangerous. The invention teaches that the risks are substantially reduced if theophylline is administered in combinations with adequate quantities of appropriate vitamin B6. Toxaemia of pregnancy is a typical example. In the toxaemic placenta both PNP oxidase and PL kinase activities may be as much as 50% lower than in normal placentas. Theophylline would therefore further compromise fetal vitamin B6 supplies from the mother by further lowering the already low PL kinase activity. The administration of high bolus doses of PN under these circumstances will predictably further complicate matters by affording yet another source of competition for the already compromised kinase enzyme system. Erratic results obtained in the treatment and prevention of pregnancy toxaemia by the use of high bolus doses of PN can therefore now be fully understood for the first time.

In the light of the aforegoing the combined administration in obstructive airways therapy of theophylline and vitamin B6, in particular PN, produces major beneficial effects and advantages which could not be foreseen or expected in the light of the existing state of the art.

Instructions may be provided with the composition for the use thereof in the treatment or prophylaxis of obstructive airways disease, in particular bronchial asthma. These instructions may be in written or printed form, e.g. included in or applied to a package for the composition.

The normal functioning (perfused) liver is the main source of plasma PLP and PL. However, this function may be impaired or delayed in serious disease.

In acute cases of asthmatic attack the desired treatment may comprise injection of a liquid formulation comprising theophylline or a physiologically acceptable base addition salt thereof. In such cases, improved, rapid counteraction of the deleterious effects (as referred to hereinbefore) of theophylline, resulting from a lowering in the PLP concentration of the plasma, and a more rapid synergistic effect may be achieved by at the same time or successively injecting or infusing the patient with vitamin B6, wholly or partially but preferably wholly in the form of PL, rather than PN, or in a proportion preferably of the order of not less than PL:PN = 1:1, the vitamin B6 preferably containing in addition zinc ions.

The above is in direct conflict with state of the art knowledge according to which (Reynolds : Am. J. Clin. Nutr. 1986, 43, 639) theophylline does not react with PLP and therefore does not lower P-PLP. Keniston also stated this explicitly at the Third International Congress on Vitamin B6 in August 1987. There thus existed a prejudice in the art which the invention had to overcome.

Therefore, for the treatment of acute obstructive respiratory failure, e.g. of acute asthma a particular embodiment of the invention is adapted for injection or infusion, the vitamin B6 being present wholly or in part in the form of pyridoxal (PL) or physiologically acceptable complex or addition salt thereof which rapidly releases PL in vivo, preferably as well as zinc ions, in the form of a physiologically acceptable zinc salt, for example zinc chloride, or as a zinc-PL complex. To prolong its shelf-life, the liquid pharmaceutical composition in accordance with the invention preferably comprises in addition an antioxidant, for example ascorbic acid.

When asthma patients are also given other vitamin B6 antagonistic drugs in an emergency situation, the co-administration intravenously or by injection of vitamin B6 in the form of PL and preferably zinc ions may make the difference for the patient between survival and death. The same may apply in the case of an asthma patient on theophylline being involved in a serious accident, such as a car crash, leading to severe physiological shock.

Based on the above findings the invention also teaches as an important preferred feature that the vitamin B6 is present in the product in a slow-release or sustained release form. This feature can be applied in combination with all xanthines, even e.g. aminophylline, which are subject to the same newly discovered mechanism of PL-kinase inhibition. However, because of the above-described chemical reactions between PL and ethylene diamino derivatives of theophylline, higher dosage rates e.g. double dosage of vitamin B6 should be employed if the xanthinine is in the form of an ethylene diamino derivative, egg. aminophylline or a piperazine acetate complex.

Thus according to a further aspect of the present invention a pharmaceutical product as set out in the opening paragraph is provided, characterised by a combination as dosage units of the xanthine and vitamin B6, at least the latter being in a slow-release or sustained-release form in an a mount effective to supplement PLP in cells and plasma.

The only way to increase the intracellular substrate PL concentration effectively is to maintain moderately raised extracellular (plasma) PL levels over long periods (24 h daily) and the only effective way of doing this is by means of slow release administration since cellular PL uptake is a saturable process. Because of the small t1/2 (40-60 min) value of PL large plasma PL fluctuations result from daily bolus doses, e.g. after 100 mg PN bolus, excessive P-PL levels (4000 nM) are reached which may cause toxicity. Physiologically, provision is made for a constant "slow release" supply of PL by means of the erythrocyte (RBC) mechanism after physiological doses of DN. Only a small percentage of an oral PN supplement appears in plasma as PLP, the remainder being metabolised in the liver and RBC to PL + PLP from which source plasma PL is gradually replenished. This follows directly from our finding that RBC-PLP values rise rapidly after an oral 10 mg PN dose to peak after 90 min thereafter rapidly declining again biexponentially to reach pre-supplementation values after only 2-3h. RBC-PLP therefore serves as a slow release source of plasma PL in this way. The operation of this process even at low (nutritional) levels of PN intake, illustrates the biological importance of the slow release principle. Intracellular PLP values increase with increasing extracellular PL values up to a value of 600 nM extracellular PL concentration. At higher extracellular values, no further rise occurs in intracellular PLP. We have demonstrated that, after an oral bolus dose of 100 mg PN (often given to patients), plasma PL values rise to very high levels approaching 4000 nM. These are obviously useless and wasteful from the point of view of intracellular PLP requirements and may be the cause of toxicity reported after high bolus doses of PN.

As explained above, it was previously assumed that the mechanism by means of which theophylline depresses plasma PLP values is based on a chemical reaction between PLP and theophylline leading to a Schiff base type of condensation product. Alternatively, it was suggested that theophylline might act by displacing PLP from protein binding sites. In both these cases no specific advantage would be attached to having the vitamin B6 delivered in a slow-release form as taught by the invention.

More specifically the vitamin B6 is present as pyridoxine (PN), in a slow- or sustained-release form. Preferably the xanthine, preferably theophylline, is also present in a slow-release or sustained release form. For example, the product comprises a slow-release form of PN and a slow or sustained-release form of theophylline in a single oral capsule. The following relevant experimental facts, now surprisingly found by us, confirm and stress the advantage of supplementing PN in slow-release form combined with theophylline in the same preparation or separately, preferably also in a slow-release form:-
a) The fact that theophylline specifically supresses the enzyme PL-kinase. Sustained low levels of theophylline will have less of an enzyme suppressing effect than massive bolus doses.
b) The modest affinity that the enzyme PL-kinase has for its substrate PL. This implies that lowered intracellular PL levels such as may occur in disease, including asthma, will adversely affect production of intracellular PLP. Maintenance of moderately elevated intracellular PL levels over longer periods is clearly advantageous.
c) We have demonstrated that very low doses of PN (0.02 mg/kg) in humans have a profound influence on vitamin B6 dependent intracellular pathways without affecting extracellular PL and PLP levels materially.

Thus a female volunteer was treated with theophylline in conventional doses to maintain her theophylline blood levels within the therapeutic range (10 - 20 mg/l) for 5 weeks. At the end of that period her xanthurenic acid (XA) 24 h excretion was 12,524 mg/24 h after a 2g Tryptophane load. She was then treated for 1 week with 1,0 mg PN daily (0,02 mg/kg (day) and her XA excretion dropped to 2,17 mg/24h. These results indicate that a special mechanism exists which operates very effectively at low PN intake levels to ensure intracellular "trapping" of available PN or PL. This same mechanism will ensure optimal PN utilisation in slow-release formulations of PN.

The most efficient pharmaceutical PN formulation for maintaining adequate vital intracellular PLP levels depends on the nature of the process by which the cells take up extracellularly available PL. This process is on the one hand saturable as indicated and on the other hand surprisingly sufficient in the lower dosage ranges. These two facts clearly establish the advantage of slow-release formulations.

We have also demonstrated in human patients that the lowered vitamin B6 status in human patients is not only of analytical significance but has indeed metabolic consequences. Three healthy human volunteers (average XA excretion/24h after a 2g Tryptophane load: 6,57 mg) were treated for 5 weeks with theophylline, maintaining their theophylline blood levels within the therapeutic range. At the end of this period another Tryptophane load test was performd (Av. XA excretion : 16,13 mg) confirming that the lowered PLP levels these patients had at that stage were indeed metabolically significant. After these patients were then treated with vitamin B6, the XA excretion returned to normal levels.

Following our substantive investigations, including the aforegoing findings, we believe that we can now explain at least part of the mechanism on which the present invention may be based. However, the invention is not to be limited to such explanation or be dependant in any way on the correctness of the mechanism herein postulated.

### Example 1

### Capsule

Oral capsules are prepared each containing 300 mg theophylline, 100 mg pyridoxine hydrochloride, 5 mg riboflavine, 0,05 mg hydroxy cobalamine, 1,0 mg folic acid, 20,0 mg zinc chloride.

The theophylline is converted with known additives and excipients into slow release pellets or granules. Similarly the remaining ingredients together with known galenic excipients and additives are converted into slow release pellets or granules. The two kinds of granulate or pellets are mixed in the required proportions and are filled into capsules.

The slow release effect is attained by diffusion through a micro dialysis membrane. For that purpose each pellet or granule of active constituence is coated with a membrane of controlled permeability which, in a manner known per se determines the rate of liberation. Suitable coating materials for producing the coating membrane are commercially available for that purpose, e.g. specialise lacquers based on acrylic acid ester-methacrylic acid ester copolymers, usually substituted with quaternary amino groups which determine the permeability.

The dosage rate is 1 to 2 capsules per day for adults. Similar capsules containing ½ to 1/3 the above quantities are also prepared in order to allow for finer dosage level adjustment in practice.

### Example 2

### Capsules

Capsules are prepared as described in Example 1 but using aminophylline instead of theophylline. The pyridoxine content of each capsule is increased to 150 mg.

Again the dosage rate is 1 to 2 capsules per day for adults in split dosages.

### Example 3

### Solution for intravenous administration in asthma patients

a) Vitamin B6 ampoules.
   Each ampoule contains pyridoxal.HCl: 121,8 mg (= 100 mg pyridoxal)
   Pyridoxine.HCl: 121,6 mg (= 100 mg pyridoxine).
   Ampoules must be amber or otherwise dark-coloured. Prepare a solution containing 24,4 g of pyridoxal.HCl and 24,4 g of pyridoxine.HCl per litre. To this should be added 50 mg/l ascorbic acid as an antioxidant.
   Adjust pH to 5,0 - 6,0.
   Sterilise by heat and/or filtration. (Both pyridoxal and pyridoxine are stable for hours in boiling, dilute aqueous acid solutions).
   Use 5,0 ml per ampoule for freeze drying. Fill into ampoules. Protect against light.
b) Theophylline or aminophylline ampoules.
   Contents per ampoule 350 mg theophylline or aminophylline, 5 mg riboflavine, 0,05 mg hydroxy cobalamine, 1,0 mg folio acid and 20.0 mg zinc chloride.
   The above ingredients are made up in the aforesaid proportions in an aqueous solution, making due allowance for solubility requirements, sterilised by heat and/or filtration and filled into ampoules.
c) Making up infusion solution.
   The desired numbers of ampoules a) and b) in a ratio of 1:1 to 2:1, depending on desired dosage rate, are added to the desired amount of infusion solution which can be any standard infusion solution (glucose, saline, electrolytes). The contents of the freeze-dried vitamin B6 ampoules are first reconstituted in 5 ml or more of the sterile infusion solution and are then added to the bulk of the infusion solution.
   Total daily dose 1/5 to 5 ampoules (per 70 kg) per day vitamin 86 and 1/3 to 1 ampoule per day of the ampoule containing theophylline.
   Typical rates of infusion are such that 10 to 20 mg per 70 kg per hour of the combined pyridoxal and pyridoxine ("total vitamin B6 activity") is given. Do not exceed 100 mg/70 kg per hour or more than a total dose of 500 mg/day/70 kg of total vitamin B6 activity.

### Example 4

### Preparation for parenteral administration

The contents of ampoules a) and b) in Example 3 are combined in a single solution of 20 ml, sterilised and divided into 4 ampoules of 5 mg each for IV, IM or SC administration. The administration takes place in suitably divided dosages spread over a day.

### Example 5

### Suppositories

The ingredients as set out in Example 1 are incorporated in a suppository composition known per se and cast into suppositories, each containing half the amount of active ingredient of the capsules in Example 1. The dosage rate is 3 suppositories per day for adults spread evenly over the day.

### Example 6

### Tablets

The granules described in Example 1 are pressed into tablets and are sugar-coated in a manner known per se. Each tablet contains half the amount of active ingredients of the capsules in Example 1, and the tablets are taken at a rate of 2 to 6 tablets per day for adults spread over 24 hours.

### Example 7

### Lozenges

The ingredients in the proportion stated in Example 1 are compounded in the form of sublingual lozenges, each lozenge containing 1/3 the amount of active ingredients stated in Example 1. The lozenges are taken at a rate of 3 to 8 lozenges per day spread evenly over the day.

### Example 8

### Syrups or suspensions - adult or pediatric

The ingredients in the ratio set out in Example 1 are made up in syrup or suspension form, suitably buffered and preserved and containing conventional thickening and sweetening agents. The active ingredients are present in a concentration that dosage rates equivalent to those in Example 1 are attained if the medication is taken at regular intervals spread over the day in conventionally sized measures, neat or diluted in water.

The above examples when read together with the preceding general disclosure and with the claims which are part of that disclosure will enable the person skilled in the art to practise the various aspects of the invention on an industrial scale in the manufacture of pharmaceuticals for human and veterinary use.

The invention as described in the aforegoing, although primarily intended for obstructive air passage therapy, e,g. emphysema, chronic bronchitis and in particular asthma therapy in humans, can also be applied in the veterinary field to animals which are inclined to suffer from asthma, asthma-like conditions or other bronchial conditions associated with respiratory failure. The term "pharmaceutical products" or "pharmaceutical preparation" is to be so understood.

## Claims

1. A pharmaceutical product comprising a xanthine type drug for use in obstructive air passage and in particular bronchial asthma therapy characterised by a combination as dosage units of theophylline in its non-ethylene di-aminated form and vitamin B6 in a form and amount effective to supplement PLP in cells and plasma, wherein the weight ratio of the theophylline and the vitamin B6 in the dosage units is between 2 and 120.

2. A product as claimed in claim 1, wherein the vitamin B6 is provided in combination with riboflavin and zinc ions.

3. A product as claimed in claim 1 or 2, characterised in that the theophylline and the vitamin B6 are combined in a single dosage form.

4. A product as claimed in any one of claims 1 to 3 for oral administration.

5. A product as claimed in any one of claims 1 to 4, characterised in that the vitamin B6 is present in a slow-release or sustained-release form.

6. A product as claimed in claim 5, wherein the vitamin B6 is present as pyridoxine (PN), in a slow-release or sustained release form.

7. A product as claimed in any one of claims 1 to 6, comprising a slow-release form of PN and a slow- or sustained-release form of theophylline in a single oral dosage form.

8. A product as claimed in any one of claims 1 to 7, comprising a daily dosage rate for human adults of 5 to 500 mg, preferably 10 to 200 mg of PN and 50 to 600 mg, preferably 100 to 500 mg of theophylline; or correspondingly reduced dosage rates for children.

9. A product as claimed in claim 8 which further comprise a daily supplement of 5 to 100 mg zinc as ions or as a coordination complex with vitamin B6.

10. A product as claimed in claim 8 or 9 which further comprises a daily supplement of 1 mg to 20 mg, preferably 3 - 10 mg riboflavin.

11. A product as claimed in any one of claims 1 to 10 for the treatment of acute obstructive respiratory failure, in particular acute asthma characterised in that it is adapted for injection or infusion, the vitamin B6 being present wholly or in part in the form of pyridoxal (PL) or physiologically acceptable complex or addition salt thereof which rapidly releases PL in vivo, optionally in combination with an antioxidant.

12. A product as claimed in claim 11 characterised in that it comprises in addition zinc ions, optionally as a complex of vitamin B6.

13. A pharmaceutical product comprising a xanthine type drug for use in obstructive air passage therapy, in particular asthma therapy characterised by a combination as dosage units of the xanthine and vitamin B6, at least the latter in a slow-release or sustained-release form in an amount effective to supplement PLP in cells and plasma.

14. The use of a combination or theophylline in a non-ethylene diaminated form and vitamin B6 in the preparation of medicines for the treatment or prophylaxis of obstructive air passage diseases, in particular bronchial asthma.

15. The use of a combination of a xanthine and vitamin B6 in the preparation of medicines containing at least the vitamin B6 and preferably also the xanthine in a slow release or sustained release form for the treatment or prophylaxis of obstructive air passage diseases, in particular bronchial asthma.

16. A pharmaceutical product comprising a xanthine type drug for use in obstructive air passage and in particular bronchial asthma therapy characterised by a combination as dosage units of the xanthine type drug and vitamin B6 in a form and amount effective to supplement PLP in cells and plasma, wherein the weight ratio of the xanthine type drug and the vitamin B6 in the dosage units is between 2 and 120.

17. A product as claimed in claim 16, characterised in that the vitamin B6 is at least in part provided in the form of pyridoxal.

18. A product as claimed in claim 17, wherein the xanthine type drug is theophylline.

## Patentansprüche

1. Ein ein xanthin-artiges Arzneimittel enthaltendes Produkt zur Verwendung in der Behandlung obstruktiver Luftwegserkrankungen und insbesondere Bronchialasthma, gekennzeichnet durch eine Kombination in Form von Dosiereinheiten von Theophyllin in seiner nicht-äthylendiaminierten Form, sowie Vitamin B6 in einer Form und Menge, die zur Anhebung von PLP in Zellen und Plasma wirksam ist, wobei das Gewichtsverhältnis von Theophyllin und dem Vitamin B6 in den Dosierungseinheiten zwischen 2 und 120 beträgt.

2. Produkt gemäß Anspruch 1, worin das Vitamin B6 in Kombination mit Riboflavin und Zinkionen vorliegt.

3. Produkt gemäß Anspruch 1 oder 2, dadurch gekennzeichnet, daß das Theophyllin und das Vitamin B6 in einer einzigen Dosierungsform kombiniert vorliegen.

4. Produkt gemäß einem der Ansprüche 1 bis 3 für die orale Verabreichung.

5. Produkt gemäß einem der Ansprüche 1 bis 4 , dadurch gekennzeichnet, daß das Vitamin B6 als verzögerte, bzw. Dauerfreisetzungs(Retard)-Formulierung vorliegt.

6. Produkt gemäß Anspruch 5, worin das Vitamin B6 in Form von Pyridoxin (PN) in einer verzögerten, bzw. Dauerfreisetzungs(Retard)-Formulierung vorliegt.

7. Produkt gemäß einem der Ansprüche 1 bis 6, welches eine verzögerte Freisetzungsformulierung von PN und eine verzögerte, bzw. Dauerfreisetzungs(Retard)-Formulierung von Theophyllin in einer einzigen oralen Verabreichungsform beinhaltet.

8. Produkt gemäß einem der Ansprüche 1 bis 7, welches eine tägliche Dosierung für menschliche Erwachsene von 5 bis 500 mg, vorzugsweise 10 bis 200 mg an PN und 50 bis 600 mg, vorzugsweise 100 bis 500 mg an Theophyllin beinhaltet, oder eine entsprechend geringere Dosierung für Kinder.

9. Produkt gemäß Anspruch 8, welches ferner eine tägliche Zusatzverabreichung von 5 bis 100 mg Zink als Ionen oder als Koordinationskomplex mit Vitamin B6 beinhaltet.

10. Produkt gemäß Anspruch 8 oder 9, welches ferner eine tägliche Zusatzverabreichung von 1 bis 20 mg, vorzugsweise 3 bis 10 mg Riboflavin beinhaltet.

11. Produkt gemäß einem der Ansprüche 1 bis 10 führ die Behandlung von akuter obstruktiver Atemnot, insbesondere akutem Asthma, dadurch gekennzeichnet, daß es für die Einspritzung bzw. Infusion formuliert ist, wobei das Vitamin B6 ganz oder teilweise als Pyridoxal (PL) oder dessen physiologisch akzeptablen Komplex oder Additionssalz vorliegt, welches schnell PL im Körper freisetzt, ggf. in Kombination mit einem Antioxydanten.

12. Produkt gemäß Anspruch 11, dadurch gekennzeichnet, daß es außerdem Zinkionen, ggf. als Komplex mit Vitamin B6, enthält.

13. Ein ein xanthin-artiges Arzneimittel enthaltendes Produkt zur Verwendung in der Therapie obstruktiver Luftwegserkrankungen, insbesondere Asthmatherapie, gekennzeichnet durch eine Kombination als Dosiereinheiten von Xanthin und von Vitamin B6, wobei wenigstens Letzteres als verzögert, bzw. dauerfreisetzende (Retard)-Form in einer Menge vorliegt, die zur Anhebung des PLP-Spiegels in Zellen und Plasma geeignet ist.

14. Verwendung einer Kombination von Theophyllin in seiner nicht-äthylendiaminierten Form und Vitamin B6 für die Herstellung von Arzneimitteln zur Behandlung oder Prophylaxe von obstruktiven Luftwegserkrankungen, insbesondere Bronchialasthma.

15. Die Verwendung einer Kombination eines Xanthins und Vitamin B6 für die Herstellung von Arzneimitteln, worin wenigstens das Vitamin B6 und vorzugsweise auch das Xanthin als verzögert, bzw. dauerfreisetzende (Retard)-Form vorgesehen ist, für die Behandlung oder Prophylaxe von obstruktiven Luftwegserkrankungen, insbesondere Bronchialasthma.

16. Pharmazeutisches ein xanthin-artiges Arzneimittel enthaltendes Produkt für die Behandlung von obstruktiven Luftwegserkrankungen und insbesondere für die Bronchialasthmatherapie, gekennzeichnet durch eine Kombination als Dosiereinheiten des xanthin-artigen Arzneimittels und Vitamin B6 in einer Form und Menge, die zur Anhebung des PLP-Spiegels in Zellen und Plasma geeignet ist, worin das Gewichtsverhältnis des xanthin-artigen Arzneimittels und des Vitamins B6 in den Dosiereinheiten zwischen 2 und 120 beträgt.

17. Produkt gemäß Anspruch 16, dadurch gekennzeichnet, daß das Vitamin B6 wenigstens teilweise als Pyridoxal vorliegt.

18. Produkt gemäß Anspruch 17, worin das xanthin-artige Arzneimittel Theophyllin ist.

## Revendications

1. Produit pharmaceutique comprenant une substance médicamenteuse du type xanthine à utiliser pour le traitement thérapeutique des obstructions des voies respiratoires et, plus particulièrement, de l'asthme bronchique, caractérisé par une combinaison, sous forme d'unités de dosage, de théophylline sous sa forme diaminée non éthylénique et de vitamine B6 sous une forme et en une quantité efficace pour compléter le PLP dans les cellules et le plasma, dans lequel le rapport pondéral de la théophylline et de la vitamine B6 varie de 2 à 120 dans les unités de dosage.

2. Produit suivant la revendication 1, caractérisé en ce que la vitamine B6 est fournie en combinaison avec de la riboflavine et des ions zinc.

3. Produit suivant la revendication 1 ou 2, caractérisé en ce que la théophylline et la vitamine B6 sont combinées en une forme de dosage unique.

4. Produit suivant l'une quelconque des revendications 1 à 3, destiné à l'administration par la voie orale.

5. Produit suivant l'une quelconque des revendications 1 à 4, caractérisé en ce que la vitamine B6 est présente sous une forme à libération lente ou à libération soutenue ou progressive.

6. Produit suivant la revendication 5, caractérisé en ce que la vitamine B6 est présente sous forme de pyridoxine (PN), dans une forme à libération lente ou soutenue ou progressive.

7. Produit suivant l'une quelconque des revendications 1 à 6, comprenant une forme à libération lente de la PN et une forme à libération lente ou soutenue ou progressive de la théophylline en une forme de dosage orale unique.

8. Produit suivant l'une quelconque des revendications 1 à 7, caractérisé en ce qu'il donne un taux de dosage quotidien pour les êtres humains adultes de 5 à 500 mg, de préférence 10 à 200 mg, de PN et de 50 à 600 mg, de préférence 100 à 500 mg, de théophylline, ou des taux de dosage réduits en correspondance pour les enfants.

9. Produit suivant la revendication 8, caractérisé en ce qu'il fournit un supplément quotidien de 5 à 10 mg de zinc sous forme d'ions, ou sous forme d'un complexe par coordinence avec la vitamine B6.

10. Produit suivant la revendication 8 ou 9, caractérisé en ce qu'il fournit un supplément quotidien de 1 mg à 20 mg, de préférence de 3 à 10 mg, de riboflavine.

11. Produit suivant l'une quelconque des revendications 1 à 10, pour le traitement des troubles respiratoires par obstruction aigus, plus particulièrement l'asthme aigu, caractérisé en ce qu'il est adapté à l'injection ou à la perfusion, la vitamine B6 étant présente en entier ou en partie sous la forme de pyridoxal (PL) ou d'un sel d'addition ou d'un complexe physiologiquement acceptable de celui-ci, qui libère rapidement le PL in vivo, éventuellement en combinaison avec une substance antioxydante.

12. Produit suivant la revendication 11, caractérisé en ce qu'il comprend en outre des ions zinc, éventuellement sous forme de complexe avec la vitamine B6.

13. Produit pharmaceutique comprenant un médicament du type xanthine destiné au traitement thérapeutique des obstructions des voies respiratoires, plus particulièrement de l'asthme, caractérisé par une combinaison, sous forme d'unités de dosage, de la xanthine et de la vitamine B6, cette dernière au moins sous une forme à libération lente ou à libération soutenue ou progressive, en une proportion efficace pour compléter le PLP dans les cellules et le plasma.

14. Emploi d'une combinaison de théophylline en une forme diaminée et non éthylénique et de la vitamine B6 dans la préparation de médicaments pour le traitement ou la prophylaxie de maladies des voies respiratoires par obstruction, plus particulièrement l'asthme bronchique.

15. Utilisation d'une combinaison de la xanthine et de la vitamine B6 pour la préparation de médicaments contenant au moins la vitamine B6 et, de préférence également la xanthine, sous une forme à libération lente ou à libération progressive ou soutenue, en vue du traitement ou de la prophylaxie de maladies des voies respiratoires par obstruction, plus particulièrement l'asthme bronchique.

16. Produit pharmaceutique comprenant un médicament du type xanthine à utiliser pour le traitement thérapeutique des obstructions des voies respiratoires, plus particulièrement de l'asthme bronchique, qui se caractérise par une combinaison, sous forme d'unités de dosage, d'un médicament de type xanthine et de vitamine B6, sous une forme et en une proportion efficace pour compléter le PLP dans les cellules et le plasma, où le rapport pondéral du médicament du type xanthine à la vitamine B6 varie de 2 à 120 dans les unités de dosage.

17. Produit suivant la revendication 16, caractérisé en ce que la vitamine B6 est fournie au moins en partie sous forme de pyridoxal.

18. Produit suivant la revendication 17, caractérisé en ce que le médicament du type xanthine est la théophylline.
